(19) **Europäisches Patentamt**
**European Patent Office**
**Office européen des brevets**

(11) Veröffentlichungsnummer: **0 222 198 B1**

(12) **EUROPÄISCHE PATENTSCHRIFT**

(45) Veröffentlichungstag der Patentschrift: **28.11.90**

(51) Int. Cl.⁵: **A 61 K 31/645, A 61 K 47/00**

(21) Anmeldenummer: **86114334.5**

(22) Anmeldetag: **16.10.86**

(54) **Erzeugnisse enthaltend Amitriptylinoxid zur parenteralen Verabreichung bei der Therapie von Depressionen.**

(30) Priorität: **31.10.85 DE 3538771**

(43) Veröffentlichungstag der Anmeldung:
**20.05.87 Patentblatt 87/21**

(45) Bekanntmachung des Hinweises auf die
Patenterteilung:
**28.11.90 Patentblatt 90/48**

(84) Benannte Vertragsstaaten:
**AT BE CH DE ES FR GB GR IT LI LU NL SE**

(56) Entgegenhaltungen:
**FR-A-2 176 739**

**ARZNEIMITTELFORSCHUNG, Band 28, Nr. 10b,
Oktober 1978, Seiten 1879-1883, Aulendorf, DE;
H.WENZL: "Special experiments on central
nervous effects of amitriptylinoxide considering
pharmacokinetic aspects"**

(73) Patentinhaber: **A. Nattermann & Cie. GmbH
Nattermannallee 1
D-5000 Köln 30 (DE)**

(72) Erfinder: **Dürr, Manfred, Dr.
Am Blauen Stein 10
D-5024 Pulheim-Dansweiler (DE)**
Erfinder: **Harhausen, Ekkehard, Dr.
Am Döhrenkamp 1
D-2401 Sarkwitz (DE)**
Erfinder: **Seidel, Jürgen, Dr.
Venloer Strasse 67
D-5024 Pulheim (DE)**
Erfinder: **Wetzig, Helmut, Dr.
Paulstrasse 46
D-5024 Pulheim-Sinnersdorf (DE)**

(74) Vertreter: **Redies, Bernd, Dr. rer. nat.
COHAUSZ & FLORACK Patentanwaltsbüro
Schumannstrasse 97 Postfach 14 01 20
D-4000 Düsseldorf 1 (DE)**

**Beschreibung**

Die Erfindung betrifft Erzeugnisse zur parenteralen Applikation des bekannten Wirkstoffes Amitriptylinoxid, die sich besonders zur intravenösen und intramuskulären Verabreichung des Wirkstoffes in der Therapie von Depressionen eignen.

Amitriptylinoxid [3-(10,11-Dihdyro-5H-dibenzo-[a,d]cyclohepten-5-yliden)-N,N-dimethyl-1-propanamin-N-oxid] ist seit längerer Zeit zur Therapie von Depressionen im Handel. Eine parenteral applizierbare Form ist bisher nicht bekannt. Obwohl die Anwendung des Wirkstoffes normalerweise oral efolgt, ist in gewissen Fällen die parenterale Anwendung vorzuziehen, da sie den unbestreitbaren Vorteil der schnelleren und zuverlässigeren Wirkung besitzt.

Es ist bekannt, daß die Löslichkeit von Amitriptylinoxid in Wasser stark pH-abhängig ist, wie die folgenden Zahlen zeigen:

Löslichkeit bei pH 7: < 0,1%
Löslichkeit bei pH 6: ~ 0,1%
Löslichkeit bei pH 5: ~ 0,2%
Löslichkeit bei pH 4: ~ 1%
Löslichkeit bei pH ≤ 3,5: > 2,5%.

Diese Zahlen beziehen sich auf Gew.-%. Eine brauchbare Wirkstoffkonzentration von 2,5 Gew.-% ist demnach erste bei einem pH ≤ 3,5 erreicht. Es ist aber weiter bekannt, daß derart starke saure Lösungen zu erheblichen lokalen Unverträglichkeiten bei intravenöser Applikation führen. Wegen der Erwünschtheit der parenteralen Applikation des bekannten Wirkstoffes Amitriptylinoxid war es Aufbage der Erfindung, Stoffgemische zu finden, die die Applikation des Wirkstoffes Amitriptylinoxid auf parenteralem Wege ermöglichen, die Wirksamkeit dieses Wirkstoffes nicht beeinflussen und selbst keine nachteiligen physiologischen Wirkungen zeigen.

Überraschenderweise wurde gefunden, daß bei Kombination einer sauren, vorzugsweise einer salzsauren Lösung von Amitriptylinoxid mit einem pH von ≤ 4,5, die eine brauchbare Wirkstoffkonzentration ergibt, mit einer etwa 0,1 bis etwa 3%-igen Pufferlösung, vorzugsweise einer Lösung von Tris-(hydroxymethyl)aminomethan, in einer solchen Menge daß die Gesamtlösung neutralisiert ist und auf einen pH von 7 kommt, nicht bewirkt, daß der Wirkstoff Amitriptylinoxid ausfällt, obwohl eine spontane Ausfällung zu erwarten gewesen wäre, da, wie oben ausgeführt, die Löslichkeit von Amitriptylinoxid bei einem pH von 7 kleiner als 0,1 Gew.-% ist. Die auf diese Weise erhaltenen Lösungen sind ca. 1 bis 4 Stunden klar, also metastabil, und sind zur parenteralen Applikation, insbesondere zur intravenösen oder intramuskulären Injektion geeignet.

Gegenstand der Erfindung sind somit Erzeugnisse bestehend aus einer ersten, mindestens 0,5—5 Gew.-% Amitriptylinoxid enthaltenden sauren Lösung in physiologischer Natriumchloridlösung (= 0,8 Gew-% Lösung von Natriumchlorid in Wasser) mit einem pH von ≤ 4,5 und einer zweiten, eine Puffersubstanz enthaltenden Lösung ebenfalls in physiologischer Natriumchloridlösung in solcher Menge, das die Säure in der ersten Lösung durch die Zugabe der zweiten Lösung neutralisiert wird, als Kombinationspräparat zur gleichzeitigen parenteralen Verabreichung als neutalisiertes Produkt bei der Therapie von Depressionen.

Vorzugsweise sind Gegenstand der Erfindung Erzeugnisse bestehend aus einer ersten, maximal 2,5 Gew.-% Amitriptylinoxid enthaltenden, salzsauren Lösung in physiologischer Natriumchloridlösung (= 0.8 Gew.-% Lösung von Natriumchlorid in Wasser) mit einem pH 3,5 und einer zweiten, etwa 0,55 bis etwa 1,7% Tris-(hydroxymethyl)-aminomethan enthaltenden Lösung ebenfalls in physiologischer Natriumchloridlösung in solcher Menge, daß die Salzsäure in der ersten Lösung durch die Zugabe der zweiten Lösung neutralisiert wird, als Kombinationspraparat zur gleichzeitigen parenteralen Verabreichung als neutralisiertes Produkt bei der Therapie von Depressionen.

Gegenstand der Erfindung ist auch ein Verfahren zur Herstellung der erfindungsgemäßen Erzeugnisse, das dadurch gekennzeichnet ist, daß so viel Amitriptylinoxid in physiologischer Natriumchloridlösung supendiert wird und soviel Säure, vorzugsweise Salzsäure, zugegeben wird, daß die resultierende Lösung einen pH von < 4,5 hat und mindestens 0,5—5 Gew.-% Amitriptylinoxid enthält, das so erhaltene Gemisch bis zur Lösung des Amitriptylinoxids gerührt und die Lösung unter Bilding einer ersten Lösung membranfiltriert wird, eine getrennte zweite etwa 0,1 bis etwa 3%-ige Pufferlösung, vorzugsweise Lösung von Tris-(hydroxymethyl)-aminomethan, besonders bevorzugt etwa 0,55 bis etwa 1,7% Puffer enthaltende Lösung, ebenfalls in physiologischer Natriumchloridlösung mit soviel Pufferkapazität geschaffen wird, die genügt, um beim Vermischen der ersten mit der zweiten Lösung eine neutrale Gesamtlösung zu geben, und die erste Lösung und die zweite Lösung in getrennten Behältern im Erzeugnis vorgesehen werden und das Erzeugnis mit Hilfsmitteln versehen wird, die es gestatten und gewährleisten, daß die beiden Lösungen vor der parenteralen Applikation vermischt und gemeinsam als neutrale Gesamtlösung verabreicht werden Können.

Eine zur intravenösen Injektion geeignete Gesamtlösung erhält man zum Beispiel, wenn man 2 bis 4 ml einer Suspension des Wirkstoffes in physiologischer Kochsalzlösung mit zur Erzeilung des pH 3,5 notwendigen Menge Salzsäure bei mindesten 0,5—2,5 Gew.-% Wirkstoffgehalt unter Bildung der ersten Lösung vermischt, die zweite Lösung der genügenden Menge Tris-(hydroxymethyl)-aminomethan in 3 bis 6 ml physiologischer Kochsalzlösung bildet und die erste und die zweite Lösung vermischt. Die Gesamtlösung ist ca. 4 Stunden beständig. Eine zur intramuskulären Injektion brauchbare Gesamtlösung erhält man, wenn man 2 bis 4 mol Wirkstofflösung in physiologischer Kochsalzlösung mit der zur Erzeilung von pH 3,5 notwendigen Menge Salzsäure bei mindestens 0,5—2,5 Gew.-% Wirkstoffgehalt vermischt und so die erste Lösung bildet, die zweite Lösung durch Lösung einer genugenden Menge Tris-(hydroxymethyl)-aminomethan in 1 bis 2 ml physiologischer Kochsalzlösung bildet und die erste und die zweite Lösung vermischt. Die Stabilität der Gesamtlöung beträgt ca. 1 Stunde.

Die so erhaltenen parenteral applizierbaren Lösungen des Amitriptylinoxids dienen zur Therapie von Depressionen in denjenigen Fällen, in denen die parenterale Anwendung wegen der schnelleren und zuverlässigeren Wirkung vorteilhaft und daher erwünscht ist.

Die gute Verträglichkeit der aus den erfindungsgemäßen Erzeugnissen hergestellten parenteral applizierbaren Lösungen zeigt sich in folgenden Testergebnissen:

Die Prüfung erfolgte jeweils an 5 männlichen und 5 weiblichen Wistar-Ratten mit einem Anfangsgewicht von 120—140 g. Die Applikation wurde über eine Schwanzvene vorgenommen und erfolgte an saufeinanderfolgenden Tagen einmal täglich, gefolgt von einer 7-tägigen Nachbeobachtungszeit. Als Dosierung wurde die toxikologisch noch vertretbare Dosierung von 15 mg/kg Amitriptylinoxid gewählt. Die Amitriptylinoxid-Injektionslösungen wurden vor der Applikation entweder mit physiologischer Natriumchloridlösung oder mit 3 ml Tris-(hydroxymethyl)aminomethan-Lösung und physiologischer Natriumchloridlösung verdünnt, um eine Applikationsvolumen von 6 ml/kg Körpergewicht zu erreichen.

In der Versuchsreihe wurden die Veränderungen an den Schwanzgefäßen fotographisch dokumentiert. Die aufnahmen erfolgten am 5. Tag der Injektionsbehandlung und nach einer 7-tägigen Nachbeobachtungszeit. Die Applikation unverdünnter, saurer Amitriptylinoxid-Lösungen an Ratten führt zu starken, lokalen Gefäßveränderungen, die nach 3 Tragen eine Applikation nicht mehr zulassen und zu Nekrosen im Applikationsgebiet führen. Ausgangslösungen mit dem pH-Wert < 4,5 führten auch nach Verdünnung mit physiologischer Natriumchloridlösung zu schlechter Gefäßverträglichkeit.

Demgegenüber ergab die Amitriptylinoxid-Injektionslösung von pH 7, die vor der Applikation mit tris-(hydroxymethyl)aminomethan-Lösung und physiologischer Natriumchloridlösung versetzt und neutralisiert wurde, eine gute Gefäßverträglichkeit bei intravenöser Applikation an 5 aufeinanderfolgenden Tagen. Innerhalb einer 7-tägigen Nachbeobachtungszeit wurden makroskopisch keine Gefäßschäden dokumentiert.

Die Herstellung der erfindungsgemäßen parenteral applizierbaren Produkte von Amitriptylinoxid werden durch die folgenden Beispiele nähere erläutert.

## Beispiel 1

2,5 g Amitriptylinoxid-dihydrat werden in 80 ml physiologischer Natriumchloridlösung suspendiert. Durch Zugabe von 6,6 ml 1—N Salzsäure zu der Suspension wird eine klare Lösung (pH-Wert 3,5) erhalten, die auf das Endvolumen von 100 ml mit physiologischer Natriumchloridlösung aufgefüllt und membranfiltriert (0,2 μ) wird. In einem getrennten Behälter wird eine 0,55%-ige Lösung von Tris-(hydroxymethyl)-aminomethan-Lösung in physiologischer Natriumchloridlösung in solcher Menge vorgesehen, die genügt, beim Vermischen mit der ersten Lösung eine insgesamt neuturalisierte Gesamtlösung zur erzeugen. Die Stabilität der Gesamtlösung beträgt ca. 1 bis 4 Stunden.

## Beispiel 2

2,0 ml der nach Beispiel 1 hergestellten salzsauren 2,5%-igen Amitriptylinoxid-dihydrat-Lösung werden mit 3,0 ml der 0,55%-igen Tris-(hydroxymethyl)-aminomethan-Lösung des Beispiels 1 gemischt. Die Stabilität der Gesamtmischung beträgt ca. 4 Stunden.

## Beispiel 3

2,4 ml der nach Beispiel 1 hergestellten salzsauren 2,5%-igen Amitriptylinoxid-dihydrat-Lösung werden mit 3,6 ml der im Beispiel 1 beschriebenen 0,55/-igen Tris-(hydroxymethyl)-aminomethan-Lösung gemischt. Die Stabilität der Gesamtmischung beträgt ca. 4 Stunden.

## Beispiel 4

3,0 ml der nach Beispiel 1 hergestellten salzsauren 2,0%-igen Amitriptylinoxid-dihydrat-Lösung werden mit 3,0 ml der 0,66%-igen Tris-(hydroxymethyl)-aminomethan-Lösung gemischt. Die Stabilität der Gesamtlösung beträgt ca. 4 Stunden.

## Beispiel 5

2,0 ml der nach Beispiel 1 hergestellten salzsauren 2,5%-igen Amitriptylinoxid-dihydrat-Lösung werden mit 1,0 ml einer 1,7%-igen Tris-(hydroxymethyl)-aminomethan-Lösung gemischt. Die Stabilität der Gesamtlösung beträgt ca. 1 Stunde.

**Patentansprüche für die Vertragsstaaten: BE CH DE ES FR GB IT LI LU NL SE GR**

1. Erzeugnisse bestehend aus einer ersten, mindestens 0,5—5 Gew.-% Amitriptylinoxid enthaltenden sauren Lösung in physiologischer Natriumchloridlösung mit pH von ≤ 4,5 und einer zweiten, etwa 0,1—3%-igen Pufferlösung ebenfalls in physiologischer Natriumchloridlösung in solcher Menge, das die Säure in der ersten Lösung bei Zugabe der zweiten Lösung neutralisiert wird, in getrennt lagerfähiger Form und einem Mittel zur Vereinigung der getrennt gelagerten ersten und der zweiten Lösung kurz vor der Verabreichung als Kombinationspräparat zur gleichzeitigen parenteralen Verabreichung als kombiniertes, neutralisiertes Produkt bei der Therapie von Depressionen.

2. Erzeugnisse bestehend aus einer ersten, mindestens 0,5—2,5 Gew.-% Amitriptylinoxid enthaltenden salzsauren Lösung in physiologischer Natriumchloridlösung mit pH 3,5 und einer zweiten etwa 0,55 bis 1,7 Tris-(hydroxymethyl)-aminomethan enthaltenden ebenfalls in physiologischer Natriumchloridlösung in solcher Menge, daß die Salzsäure in der ersten Lösung durch Zugabe der zweiten Lösung neutralisiert wird.

3. Verfahren zur Herstellung der Erzeugnisse gemäß den Ansprüchen 1 und oder 2, dadurch gekennzeichnet, daß Amitriptylinoxid in physiologischer Natriumchloridlösung suspendiert wird und soviel Säure zugegeben wird, daß die resultierende Lösung einen pH von $\leq$ 4,5 hat und mindestens 0,5 bis zu 5,0 Gew.-% Amitriptylinoxid enthält, die so erhaltene Mischung bis zur Lösung des Amitriptylinoxids gerührt und die Lösung unter Bildung der ersten Lösung membranfiltriert wird, eine getrennte zweite Lösung der Puffersubstanz in physiologischer Natriumchloridlösung mit soviel Pufferkazapazität geschaffen wird, die genügt, um die beim Vermischen der ersten mit der zweiten Lösung eine neutrale Gesamtlösung zu geben, und die erste Lösung und die zweite Lösung in getrennten Behältern im Erzeugnis vorgesehen werden und das Erzeugnis mit Hilfsmitteln versehen wird, die es gestatten und gewährleisten, daß die beiden Lösungen vor der parenteralen Applikation vermischt und gemeinsam als neutrale Gesamtlösung verabreicht werden können.

**Patentansprüche für den Vertragsstaat: AT**

1. Verfahren zur Herstellung eines Erzeugnisses bestehend aus einer ersten, mindestens 0,5—5 Gew.-% Amitriptylinoxid enthaltenden sauren Lösung in physiologischer Natriumchloridlösung mit pH von $\leq$ 4,5 und einer zweiten, etwa 0,1—3%-igen Pufferlösung ebenfalls in physiologischer Natriumchloridlösung in solcher Menge, daß die Säure in der ersten Lösung bei Zugabe der zweiten Lösung neutralisiert wird, in getrennt lagerfähiger Form und einem Mittel zur Vereinigung der getrennt gelagerten ersten und der zweiten Lösung kurz vor der Verabreichung als Kombinationspräparat zur gleichzeitigen parenteralen Verabreichung als kombiniertes, neutralisiertes Produkt bei der Therapie von Depressionen, dadurch gekennzeichnet, daß Amitriptylinoxid in physiologischer Natriumchloridlösung suspendiert wird un soviel Säure zugegeben wird, daß die resultierende Lösung einen pH von $\leq$ 4,5 hat und mindestens 0,5 bis zu 5,0 Gew.-% Amitriptylinoxid enthält, die so erhaltene Mischung bis zur Lösung des Amitriptylinoxids gerührt und die Lösung unter Bildung der ersten Lösung membranfiltriert wird, eine getrennte zweite Lösung der Puffersubstanz in physiologischer Natriumchloridlösung mit soviel Pufferkazapazitat geschaffen wird, die genügt, um beim Vermischen der ersten mit der zweiten Lösung eine neutrale Gesamtlösung zu geben, und die erste Lösung und die zweite Lösung in getrennten Behältern im Erzeugnis vorgesehen werden und das Erzeugnis mit Hilfsmitteln versehen wird, die es gestatten und gewährleisten, daß die beiden Lösungen vor der parenteralen Applikation vermischt und gemeinsam als neutrale Gesamtlösung verabreicht werden können.

2. Verfahren gemäß Patentanspruch 1, dadurch gekennzeichnet, daß die erste Lösung mindestens 0,5 bis 2,5 Gew.-% Amitriptylinoxid enthält und einen pH von 3,5 hat und daß die zweite Lösung etwa 0,55 bis 1,7% Tris-(hydroxymethyl)-aminomethan enthält.

**Revendications pour les Etats contractants: BE CH DE ES FR GB IT LI LU NL SE GR**

1. Produits se composant d'une première solution acide constituée d'au moins 0,5 à 5 parties-poids d'oxyde d'amitriptyline dans une solution physiologique de chlorure de sodium avec un pH $\leq$ 4,5 et d'un deuxième solution-tampon d'env. 0.1 à 3% également dans une solution physiologique de chlorure de sodium dans une quantité lors de l'addition de la première solution est neutralisé lors de l'addition de la deuxième solution, sous une forme séparée apte à être stockée, et d'un produit pour la combinaison de la première et de la deuxième solution stockées séparément peu avant l'administration en tant que préparation combinée pour l'administration parentérale momentanée simultanée comme produit neutralisé combiné lors de la thérapie des dépressions.

2. Produits se composant d'une première solution muriatée contenant au moins 0.5 à 2.5 parties-poids d'oxyde d'amitriptyline dans une solution physiologique de chlorure de sodium avec un pH de 3.5 et d'une deuxième solution contenant env. 0.55 à 1.7% de tris(hydroxymèthyl)amino-méthane également dans une solution physiologique de chlorure de sodium dans une quantité telle que l'acide muriatique de la première solution est neutralisé lors de l'addition de la deuxième solution.

3. Procédé de fabrication des produits selon la revendication 1 et/ou 2, caractérisé par le fait que l'oxye d'amitriptyline est en suspension dans la solution physiologique de chlorure de sodium et que de l'cide est ajouté dans une quantité permettant d'obentir une solution avec un pH $\leq$ 4.5 et qui contient au moins 0.5 jusqu'à 5.0 parties-poids d'oxyde d'amitriptyline, que le mélange obtenue est mélangé jusqu'à la dissolution de l'oxyde d'amitriptyline, que la solution est filtrée par une membrane sous formation de la première solution, qu'une deuxième solution séparée de la substance-tampon est créée dans la solution physiologique de chlorure de sodium avec une capacité-tampon qui suffit pour obtenir une solution globale neutre lors du mélange de la première et de la deuxième solutions et que la première solution et la deuxième solution sont prévues dans des conteneurs séparés dans le produit et que le produit est doté d'adjuvants qui permettent et garantissent le mélange des deux solutions avant l'application parentérale et qui sont administrées conjointement sous forme d'une solution globale neutre.

**Revendications pour l'Etat contractant: AT**

1. Procédé de fabrication d'un produits se composant d'une première solution acide contenant au moins 0,5 à 5 parties-poids d'oxyde d'amitriptyline dans une solution physiologique de chlorure de sodium avec un pH ≤ 4,5 et d'une deuxième solution-tampon d'env. 0.1 à 3% également dans une solution physiologique de chlorure de sodium dans une quantité telle que l'acide de la première solution est neutralisé lors de l'addition de la deuxième solution, sous une forme séparée apte à être stockée, et d'un produit pour la combinaison de la première et de la deuxième solution stockées séparément peu avant l'administration en tant que préparation combinée pour l'administration parentérale momentanée simultanée comme produit neutralisé combiné lors de la thérapie des dépressions, caractérisé par le fait que l'oxyde d'amitriptyline est en suspension dans la solution physiologique de chlorure de sodium et que l'acide est ajouté dans une quantité permettant d'obtenir une solution avec un PH ≤ 4.5 et qui contient au moins 0.5 jusqu'à 5.0 parties-poids d'oxyde d'amitriptyline, que le mélange obtenu est mélangé jusqu'à la dissolution de l'oxyde d'amitriptyline, que la solution est filtrée par une membrane sous formation de la première solution, qu'une deuxième solution distincte de la substance-tampon est créée dans la solution de chlorure de sodium physiologique avec une capacité-tampon qui suffit pour obtenir une solution globale neutre lors du mélange de la première et de la deuxième solutions et que la première solution et la deuxième solution sond prévues dans des conteneurs séparés dans le produit et que le produit est doté d'adjuvants qui permettent et garantissent le mélange des deux solutions avant l'application parentérale et qui sont administrées conjointement sous forme d'une solution globale neutre.

2. Procédé selon la revendication 1, caractérisé par le fait que la première solution contient au moins 0.5 à 5 parties-poids d'oxyde d'amitriptyline avec un pH de 3.5 et que la deuxième solution contient env. 0.55 à 1.7 de tris-(hydroxyméthyl)-aminométhane.

**Claims for the Contracting States: BE CH DE ES FR GB IT LI LU NL SE GR**

1. Products consisting of a first acidic solution containing at least 0.5—5% by weight amitriptyline N-oxide in physiological saline solution having a pH ≤ 4.5 and a second ca. 0.1 to 3% buffer solution also in physiological saline in such quantities that the acid in the first solution is neutralized by addition of the second solution, in forms that allow separate storage and a means of combining the separately stored first and second solutions shortly before administration as combined preparation for the simultaneous, parenteral administration as a combined, neutralized preparation for the treatment of depression.

2. Products consisting of a first acidic solution containing at least 0.5—2.5% by weight amitriptyline N-oxide in physiological saline solution with pH 3.5 and a second ca. 0.55 to 1.7% tris-(hydroxymethyl)aminomethane solution in physiological saline in such quantities that the acid in the first solution is neutralized of a second solution.

3. Methods for the manufacture of the products according to claims 1 and/or 2 whereby the amitriptyline N-oxide is suspended in physiological saline solution and sufficient acid is added that the resulting solution has a pH of ≤ 4.5 and contains at least 0.5 up to 5.0% by weight amitriptyline N-oxide, the mixture so obtained is stirred until the amitriptyline N-oxide has dissolved, this solution is then membrane-filtered yielding the first solution; a separate second solution of the buffer substance in physiological saline solution with sufficient buffer capacity to yield a neutral combined solution on mixing the first solution with the second solution and the first and the second solution filled into separated containers in the product and the product equipped with means that allow and guarantee that the two solutions are mixed before parenteral administration and can be administered together as a neutral combined solution.

**Claims for the Contracting State: AT**

1. Methods for the manufacture of a product consisting of a first acidic solution containing at least 0.5—5% by weight amitriptyline N-oxide in physiological saline solution having a pH ≤ 4.5 and a second ca. 0.1 to 3% buffer solution also in physiological saline in such quantities that the acid in the first solution is neutralized by addition of the second solution, in forms that allow separate storage and a means of combining the separately stored first and second solutions shortly before administration as combined preparation for the simultaneous, parenteral administration as a combined, neutralized preparation for the treatment of depression, whereby the amitriptyline N-oxide is suspended in physiological saline solution and sufficient acid is added that the resulting solution has a pH of ≤ 4.5 and contains at least 0.5 up to 5.0% by weight amitriptyline N-oxide, the mixture so obtained is stirred until the amitriptyline N-oxide has dissolved, this solution is then membrane-filtered yielding the first solution; a separate second solution with sufficient buffer capacity to yield a neutral combined solution on mixing the first solution with the second solution and the first and the second solution filled into separated containers in the product and the product equipped with means that allow and guarantee that the two solutions are mixed before parenteral administration and can be administered together as a neutral combined solution.

2. Methods according to claim 1 whereby the first solution contains at least 0.5—2.5% by weight amitriptyline N-oxide and has pH of 3.5 and the second solution contains ca. 0.55 to 1.7% tris(hydroxymethyl)aminomethane.